# EUROPEAN PATENT APPLICATION

(11) **EP 2 292 102 A1**
(43) Date of publication of application: **09.03.2011**
(21) Application number: 09011246.7
(22) Date of filing: 02.09.2009
(51) Int. Cl.: A23L 1/00, A23L 1/304, A23P 1/04

(54) **Microcapsules containing salts for food products**

(71) Applicant: Lipofoods, S.L., 08850 Gava (ES)
(72) Inventor: Lakkis, Jamileh M., 08008 Barcelona (ES); García Anton, José Maria, 08017 Barcelona (ES)
(74) Representative: Carvajal y Urquijo, Isabel

(57) **Abstract**

The present invention is directed to a composition for delivering edible salts into food products via microcapsules comprising a protein-polysaccharide shell matrix and an edible salt-containing active material.

## Description

### BACKGROUND OF THE INVENTION

Fortification of food products with calcium has been an on-going challenge to food formulators and developers. Most calcium salts are very reactive in aqueous media especially during heat processing which often results in undesirable functional and sensory consequences such as precipitation, flocculation, sedimentation and gritty mouth-feel. Slightly-soluble or insoluble calcium salts have been successfully incorporated into select food formulations; however their wider usage has been limited by the need to grind the powders to submicron particle size. Despite its positive impact on mouthfeel, micronizing magnifies the surface area of particles which renders them more susceptible to interactions. In addition, particle size reduction processes have often been found to be very labor-intensive and cost prohibitive.

One approach to broadening the utilization of active components in foods as well as other biological systems is via microencapsulation whereby particles or molecules of minerals, flavors, fragrances or other biological or inorganic actives are entrapped in a protective matrix that can shield them from their microenvironment, thus restricting their mobility and interactions but most importantly controlling their release.

Significant progress in microcapsule designs and process technologies has been reported in the last two decades. Technologies such as fluid bed particle coating, spray-drying, freeze drying, extrusion, co-extrusion, emulsions, coacervation, as well as their combinations, have been used effectively in microencapsulating a variety of active ingredients. Despite these advances, encapsulating minerals, especially calcium, remains a challenge due to many technical reasons, in particular the high concentrations of elemental calcium required for food fortification applications. For example, current US recommended daily allowance (RDA) for calcium is 1200 mg per unit food serving for an adult compared to 11 mg zinc or 27 mg iron.

US 6468568 B1 describes an extrusion system for encapsulating calcium and other minerals by embedding in a glassy sugar matrix, using hot melt extrusion, followed by grinding to a desired particle size. Despite its high throughput and economical advantages, usage of microcapsules prepared using this process are quite limited to surface sprinkles and other low moisture applications. WO 03/095085 A1 patent claims calcium nanocapsules for food fortification applications that have been stabilized by ethylene diamine tetraacetate (EDTA) and a food grade protein. The presence of EDTA can severely limit the usage of such nanocapsules in many food systems due to regulatory concerns as well as the potential interference of this chelating agent with bioavailability of other minerals. WO 2007/062723 A1 describes the preparation of stable nano-size calcium capsules with enhanced stability and bioavailability. Despite the technical advantage of producing such particle, this invention has two major limitations. First, the low level of calcium attained in the fortified food product, and secondly, the food industry's recent concern about regulatory implications of using nanoparticles and/or disclosing their incorporation into food formulations.

Compositions comprising calcium and gelatin have been used quite extensively whereby very small amounts of calcium were incorporated to help strengthen the shell material but were not adequate for nutritional fortification applications. US 2004/0091544 A1 discloses a pharmaceutical oral formulation such as insulin that is coated onto particles of cellulose or calcium phosphate and further encapsulated in gelatin capsules or may be compressed into tablets.

JP 61-115019 A claims a pharmaceutical formulation comprising calcium phosphates in gelatin membranes. The capsules when administered orally are claimed to dissolve rapidly in the digestive tract to release the drug instantly. A capsule membrane was prepared by combining gelatin, glycerin, calcium apatite, and water.

Furthermore, microcapsules of the state of the art lack stability in aqueous media due to the decomposition of the shelf material and subsequent release of the mineral salt from the microcapsules. This lack of stability represents an important drawback for the fortification of foods with a high or moderate water content.

Thus, there remains a need for a stable delivery system for fortifying food formulations with high concentrations of calcium to meet the consumer's daily requirements yet presenting no adverse effects on the functional or sensory aspects of the finished product.

### DETAILED DESCRIPTION OF THE INVENTION

The present invention provides a stable microcapsule comprising at least one mineral salt where said mineral salt is completely insulated inside the microcapsule. In the context of the present invention, stable microcapsule means that the microcapsule is able to maintain its shape and size in both hydrophobic and/or hydrophilic media.

The stable microcapsule of the invention comprises a protein-polysaccharide shell matrix and a core, said core comprising an emulsion of an aqueous dispersion or solution of at least one mineral salt in an edible oil, said mineral salt being entrapped in the core of the microcapsule and said mineral salt being insulated from the protein-polysaccharide matrix. This assembly provides stability for the microcapsule in aqueous media and in food formulations with a high or moderate water content. The structure of the microcapsule allows the incorporation of high concentrations of those minerals and reduces any negative impact on the functional and sensory aspects of the food product.

In embodiments of the present invention, the protein of the stable microcapsule is selected, but not limited to, from the group comprising gelatin, caseins and caseinates, whey proteins, soy proteins, pea proteins, wheat proteins, corn proteins, barley proteins, egg proteins, muscle proteins, proteins from other legumes and tubers, glycoproteins such as chondroitins, glucosaminoglycans or lectins, and combinations thereof. In preferred embodiments of the invention, the protein is gelatin.

In embodiments of the present invention, the polysaccharide of the stable microcapsule is selected, but not limited to, from the group comprising agar, fructo-oligosaccharides such as inulin, starches, both modified and natural, and starch fractions including amylose and amylopectin, pectins, such as low or high methoxy pectins, alginates, such as sodium or potassium alginate, natural and synthetic gums, such as gum arabic, gellan gum, welan gum, gum tragacanth, xanthan gum, guar gum or locust bean gum, celluloses, such as carboxymethyl cellulose, hydroxypropyl cellulose, hydroxymethyl cellulose, hydroxybutyl carboxymethyl cellulose, hydroxypropylethyl cellulose or methylethyl cellulose, pullulan, carrageenans, such as alpha-, gamma-, iota-, kappa- or lambda carrageenans, and combinations thereof. In preferred embodiments of the invention, the polysaccharide is agar.

In embodiments of the present invention, the mineral salt can be a soluble or an insoluble mineral salt. Preferably, the soluble or insoluble mineral salt is a divalent mineral salt. By insoluble mineral salt we mean a salt with a solubility product preferably 10⁻⁵ or less at 25 °C in water. The divalent mineral salt which may be incorporated into the microcapsules of the invention is selected, but not limited to, from the group comprising tricalcium phosphate, calcium hydrogen phosphate, calcium dihydrogen phosphate, calcium pyrophosphate, calcium carbonate, calcium hydroxide, calcium oxide, coral calcium, calcium hydroxyapatite, calcium sulfate, calcium citrate, calcium chloride, calcium malate, calcium lactate, calcium ascorbate, calcium acetate, calcium succinate, calcium piruvate, calcium citrate-malate, calcium gluconate, calcium lactate-gluconate, calcium glubionate, calcium glycerylphosphate, calcium glucoheptonate, calcium pangamate, magnesium sulfate, magnesium phosphate, magnesium pyrophosphate, magnesium carbonate, magnesium hydroxide, magnesium oxide, magnesium chloride, magnesium citrate, trimagnesium citrate, magnesium gluconate, magnesium lactate, magnesium aspartate, magnesium levulinate, magnesium pidolate, magnesium ororate and combinations thereof. In preferred embodiments of the invention, the divalent mineral salt is tricalcium phosphate (TCP).

The amount of the mineral salt in the microcapsule of the invention ranges from 0.01 to 60% of the total weight of the microcapsule, preferably from 0.1 to 40%, and most preferably from 1 to 20%.

The edible oil of the emulsion contained in the stable microcapsule of the invention is a food grade oil, and is selected from the group comprising vegetable oils such as soybean oil, palm kernel oil, palm oil, rapeseed oil, safflower oil, flaxseed oil, evening-primrose oil, sesame oil, sunflower seed oil, canola oil, cottonseed oil, babassu oil, olive oil or algae oils, animal-derived fats such as lard, tallow, marine oils, fish oils such as sardine oil, cod liver oil, menhaden oil, salmon oil, herring oil, mackerel oil or anchovies oil, which can be fractionated, partially hydrogenated and/or interesterified, and mixtures thereof. Edible reduced- or low-calorie, or non-digestible fats, fat-substitutes, or synthetic fats, such as sucrose polyesters, naturally occurring triglycerides derived from vegetable sources, synthetic triglycerides and natural triglycerides of fatty acids, commercial mixtures of alkyl esters or triglycerides of polyunsaturated fatty acids (PUFA) such as Incromega, Promega^{®}, Omacor^{®} or Lovaza^{®}, medium chain triglycerides oil (MCT oil), and mixtures thereof, may also be used. In preferred embodiments of the invention, the edible oil is medium chain triglycerides oil (MCT oil). MCT oil may preferably have a fatty acid chain length of six to twelve carbon atoms, most preferably eight to twelve carbon atoms. The medium chain triglycerides that may be used in the present invention may include, for example, commercial mixtures such as caprylic/capric triglyceride (Crodamol^{®} GTC/C), glyceryl tricaprylate/caprate (Miglyol^{®} 810 and 812), Neobee^{®} M5, Bergabest MCT oil, corn oil, peanut oil, glycerol monooleate (Pecol^{®} FCC) or Labrafac^{®} CC, and mixtures thereof. The stable microcapsule of the present invention can optionally comprise at least one plasticizer. The plasticizer is selected, but not limited to, from the group comprising glycerin, sorbitol, maltitol, propylene glycol, polyethylene glycol, glucose, sucrose, lanolin, palmitic acid, oleic acid, stearic acid, metallic salts of fatty acids such as stearic or palmitic acid, sodium stearate, potassium stearate, glyceryl lecithin, glyceryl monostearate, propylene glycol monostearate, acetylated glycerides such as acetylated monoglyceride or glyceryl triacetate, alkyl esters of citric acid such as triethyl citrate, tributyl citrate, acetyl tributyl citrate or acetyl triethyl citrate, phtalates such as diethyl phthalate, waxes, for example, natural and synthetic waxes, animal waxes such as beeswax, vegetable waxes such as carnauba, candelilla or ouricury waxes, hydrogenated vegetable oils such as soybean oil or cottonseed oils, petroleum waxes such as polyurethane waxes, polyethylene waxes, paraffin waxes or microcrystalline waxes, mineral waxes such as ozokerite, fatty waxes, sorbitan monostearate, tallow, and mixtures thereof. In preferred embodiments of the invention, the plasticizer is glycerin. The plasticizer content can range from 0.01 to 10% of the total weight of the microcapsule, preferably from 0.05 to 8%, and most preferably from 0.1 to 5%.

To provide additional buoyancy to the microcapsule and to reduce its tendency to settle at the bottom of a given liquid preparation, the microcapsule of the present invention can optionally comprise at least one wax. The wax can be a constituent of the microcapsule matrix and/ or can be applied as an outer coating onto the surface of the microcapsule. The wax is selected from the group comprising natural and synthetic waxes, animal waxes such as beeswax, vegetable waxes such as carnauba, candelilla or ouricury waxes, hydrogenated vegetable oils such as soybean oil or cottonseed oils, petroleum waxes such as polyurethane waxes, polyethylene waxes, paraffin waxes or microcrystalline waxes, mineral waxes such as ozokerite, fatty waxes, sorbitan monostearate, tallow, and mixtures thereof.

In embodiments of the present invention, the diameter of the stable microcapsule of the invention is between 0.1 microns and 15 mm, preferably between 1 micron and 10 mm, and most preferably between 10 microns and 5 mm.

The stable microcapsule of the invention can be prepared by any of the different technologies known in the food and pharmaceutical industries: spray-drying, freeze drying, extrusion, co-extrusion, emulsions, coacervation, as well as their combinations. In a preferred embodiment, the microcapsule is prepared by a coacervation process. The present invention also provides a coacervation process for forming the stable microcapsules of the invention, whereas the microcapsules are formed and recovered in an oil bath. Droplets of the coacervate of polysaccharide, protein and the oil emulsion of the mineral salt are allowed to be formed via a gravity-controlled dripping mechanism into a cold oil bath. The cold oil bath provides additional stability to the microcapsules for their broad usage in both hydrophobic and/or hydrophilic media. The distance travelled by the droplets can be adjusted according to the viscosity of the solution and/or the desired droplet size.

The oil of the oil bath used in the coacervation process of the invention is selected from the group of food grade oils, preferably MCTs. The temperature of the oil bath is less than 30 °C, preferably less than 15 °C, and most preferably between 1 and 10 °C.

In a preferred embodiment, the coacervated microcapsule of the process of the invention are formed by mixing an aqueous phase comprising gelatin and agar with an emulsion phase comprising tricalcium phosphate aqueous dispersion in MCT oil.

The present invention also provides a fortified food product containing the stable microcapsules of the invention. The fortified food product of the invention is mineral enriched but does not show adverse organoleptic effects, such as bitter taste, chalkiness, sandiness or gritty mouthfeel.

The fortified food product of the invention contains from 0.5% to 50% by weight of said microcapsules, preferably from 1 to 40%, and most preferably from 5 to 30%.

In embodiments of the present invention, the mineral salt contained in the microcapsules of the fortified food product of the invention is a calcium mineral salt.

In embodiments of the present invention, the stable microcapsule may contain one or more food grade preservatives from natural or synthetic sources. The preservatives are selected, for example, but not limited to, from the group comprising salts of sorbic acid, sulphur dioxide, sodium bisulfite, potassium hydrogen sulfite, calcium propionate, sodium nitrate, sodium nitrite, butylated hydroxyanisole, butylated hydroxytoluene, lactic acid and lactate salts, gluconates, and mixtures thereof.

In embodiments of the present invention, an antioxidant such as, for example, but not limited to, butylated hydroxytoluene (BHT), butylated hydroxyanisole (BHA), tertiary butylhidroquinone (TBHQ), gallic acid esters such as propyl gallate, tocopherols such as vitamin E acetate, ascorbic acid, ascorbic acid salts and esters such as ascorbyl palmitate or ascorbyl acetate, and mixtures thereof, may also be included.

In embodiments of the present invention, the stable microcapsule of the invention can also include, but is not limited to, other additives commonly used in the food industry, such as colorants, flavors, flavor enhancers, taste modifiers such as sugars, sweeteners, sour agents, bitter agents, astringent agents, salty taste agents, and mixtures thereof.

The fortified food product of the present invention is appropriate for human or animal consumption, and also can be a special purpose food such as baby food, medical food, sports food, performance food or nutritional bars. In embodiments of the present invention, the fortified food product includes, but is not limited to, jelly-type confections, fruit and vegetable preserves, dairy products such as milks, infant formulas, yogurts, cheeses, butter, margarine, milk shakes or ice creams, soy products such as soy milks, soy yogurts or soy cheeses, vegetable milks such as almond or rice milks, beverages, syrups, desserts such as puddings, custards or other pasty or creamy foods, and confectionary formulations, such as chewing gum, chocolates, chewy candies, hard candies, boiled candies, caramel, fudge, jellies, gummies, gelatin candies or hard and soft panned goods. The microcapsules can be added directly to the fortified food product, or they can be provided in separate sachets. The microcapsules of the invention can be added directly to a jelly-type formulation in ready-to-eat desserts or kids' products.

In embodiments of the present invention, the fortified food product comprises 100% Recommended Daily Allowance (RDA) of mineral requirements for an adult, per unit portion of said fortified food product. The high concentration of mineral salt incorporated into the microcapsules of the invention allows the preparation of fortified food products which contain enough amount of mineral to achieve the RDA intakes for an adult. In preferred embodiments of the invention, the mineral is calcium.

The present invention also provides a method for delivering 100% Recommended Daily Allowance of mineral requirements for an adult which comprises the administration of a fortified food product which contains the microcapsules of the invention. Preferably, the mineral is calcium.

The present invention also provides a method for treating and/or preventing bone demineralization, osteoporosis or extra calcium requirements which comprises administering a sufficient amount of the fortified food product which contains the calcium microcapsules of the invention. Extra calcium requirements over the RDA are recommended, for example, to pregnant, lactating or post-menopausal women. Children also require supplementary intake of calcium.

Therefore, the present invention also provides the use of the microcapsules in the preparation of a fortified food product for the treatment or prevention of bone demineralization, osteoporosis or extra calcium requirements.

The present invention is further illustrated by the following non-limiting examples where all parts, percentages, proportions, and ratios are by weight, and all temperatures are in °C unless otherwise indicated.

### BRIEF DESCRIPTION OF THE FIGURES

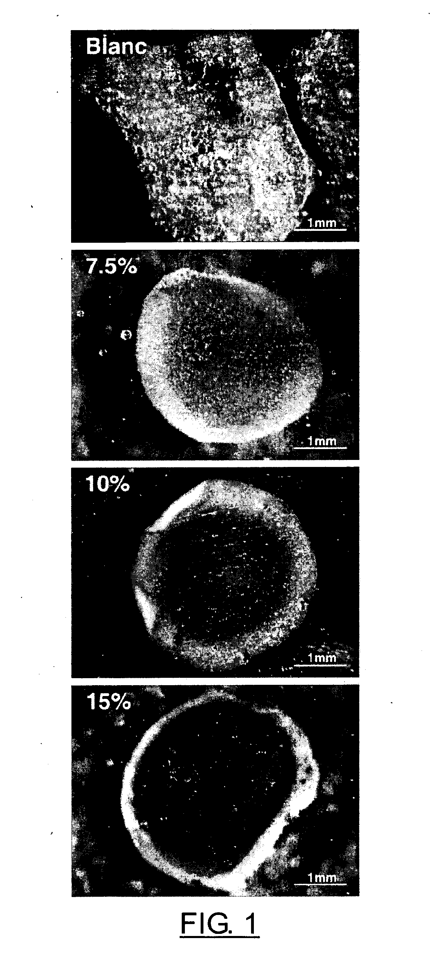

**Figure 1** shows confocal micrographs, taken with a Leica MZFLIII confocal microscope at 25x magnification, of sections of TCP-containing microcapsules where the calcium core was surrounded by the agar-gelatin coacervation shell. The calcium-specific staining (Von Kossa method) showed increased intensity with increased calcium content (0 to 15% TCP). The staining indicated that calcium was fully entrapped in the core of the microcapsule and was completely insulated from the protein-polysaccharide matrix.

### EXAMPLES

Fish gelatin type B, bloom number 240 (pH 5.71) was obtained from Lapi Gelatin, Italy and agar was supplied by Quimivita (Spain). Fine particle size (average 3 microns) tricalcium phosphate, TCP was supplied by Budenheim (Germany) and MCT oil (Bergabest brand) with C-8:C-12 ratio of 60:40 was supplied by Sternchemie Lipid technology, Germany. Fish oils, Incromega TG3322 (triglycerides, minimum 60% omega-3 PUFA) and Incromega E3322 (ethyl esters, minimum 60% omega-3 PUFA), were supplied by CRODA (United Kingdom). The beeswax was from Sonneborn Refined Products B.V. (The Netherlands). The dry jelly powder was supplied by Hacendado (Alicante, Spain). All water used in these experiments was distilled and deionized.

### Example 1. Preparation of calcium microcapsules

| **Ingredient** | **%** |
|---|---|
| Medium Chain Triglycerides oil | 8.0% |
| Tricalcium phosphate (TCP) | 5.0% |
| Glycerin | 0% |
| Agar | 1.0% |
| Gelatin type B (from fish) | 11.0% |
| Water | 75.0% |

i. Preparation of TCP emulsion: Tricalcium phosphate (10 g) was blended with water (26 g) and mixed thoroughly with MCT oil (16 g) and glycerin (2 g) using an ultra-turrax mixer to form an emulsion.
ii. Preparation of the gelatin/agar mixture: Gelatin (22 g) was hydrated in water (60 g) for 30 minutes and heated to 50 °C. Agar (2 g) was dispersed in water (62 g) and heated to 55 °C and immediately added to the hot gelatin solution and mixed thoroughly.

The TCP emulsion was added quickly to the gelatin/agar preparation followed by continuous mixing to form a homogeneous thick mass. The latter was allowed to fall dropwise via a 0.5 mm hole into a cold (4 °C) MCT oil bath. The resulting microcapsules were recovered by filtration or decantation.

### Example 2. Preparation of calcium microcapsules with glycerin as plasticizer

| **Ingredient** | **%** |
|---|---|
| Medium Chain Triglycerides oil | 8.0% |
| Tricalcium phosphate (TCP) | 5.0% |
| Glycerin | 1% |
| Agar | 1.0% |
| Gelatin type B (from fish) | 11.0% |
| Water | 74.0% |

Microcapsules were prepared in a similar manner to example 1 except for the addition of the glycerin plasticizer in the gelatin/agar preparation.

### Example 3. Preparation of fish oil microcapsules

| **Ingredient** | **%** |
|---|---|
| Fish oil (PUFA) | 8.0% |
| Tricalcium phosphate (TCP) | 5.0% |
| Glycerin | 1% |
| Agar | 1.0% |
| Gelatin type B (from fish) | 11.0% |
| Water | 74.0% |

Microcapsules were prepared in a similar manner to example 1 except for the substitution of MCT oil in the TCP emulsion by fish oil.

### Example 4. Preparation of wax-containing microcapsules

| **Ingredient** | **%** |
|---|---|
| Molten beeswax:MCT (50:50) | 8.0% |
| Tricalcium phosphate (TCP) | 5.0% |
| Glycerin | 1% |
| Agar | 1.0% |
| Gelatin type B (from fish) | 11.0% |
| Water | 74.0% |

Microcapsules were prepared in a similar manner to example 1 except for the partial substitution of some of the MCT oil in the TCP emulsion by beeswax. The coacervate mixture was held at 65 °C prior to dripping into the oil bath.

### Example 5. Preparation of wax-coated microcapsules

Microcapsules prepared using Example 1 were further coated with a thin-film of beeswax. 100 g calcium capsules were placed in a rotary pan coater and a fine spray of molten beeswax was applied onto the capsules until sufficient coating was achieved.

### Example 6. Incorporation of calcium microcapsules into a jelly-type dessert

Water (250 ml) was brought to a boil and slowly added a commercial preparation of lemon-flavored dry jelly powder (15 g). The mixture was further stirred (following manufacturer's directions) until complete dissolution of the powder. Cold water (250 ml) was added and mixing was continued until the temperature of the gelatin solution dropped to about 30 °C. Calcium-containing microcapsules of Example 1 (15 g) were added and the preparation was mixed lightly and allowed to cool at 4 °C in a refrigerator for at least 3 hours. Microcapsules of this invention were found to be stable in the jelly preparation throughout the product's shelf life (3 months).

### Example 7. Incorporation of calcium microcapsules into fruit-in-the bottom yogurt.

3 g of calcium microcapsules of Example 1 were gently mixed with 100 g of fruit preparation and pasteurized at 90 °C for 3 minutes. The pasteurized preparation was topped with yogurt and placed immediately in a 4 °C refrigerator. Stability of the preparation was checked and the calcium microcapsules were found to be intact over a period of 21 days at 4 °C.

## Claims

1. A stable microcapsule comprising a protein-polysaccharide shell matrix and a core, said core comprising an emulsion of an aqueous dispersion or solution of at least one mineral salt in an edible oil, said mineral salt being entrapped in the core of the microcapsule and said mineral salt being insulated from the protein-polysaccharide matrix.

2. A microcapsule according to Claim 1, **characterized in that** the protein of said shell matrix is selected from the group comprising gelatin, caseins and caseinates, whey proteins, proteins from legumes and tubers, soy proteins, pea proteins, wheat proteins, corn proteins, barley proteins, egg proteins, muscle proteins, glycoproteins, chondroitins, glucosaminoglycans, lectins, and combinations thereof.

3. A microcapsule according to Claim 1, **characterized in that** the polysaccharide of said shell matrix is selected from the group comprising agar, fructo-oligosaccharides, inulin, modified starches, natural starches, starch fractions, amylase, amylopectin, pectins, low methoxy pectins, high methoxy pectins, alginates, sodium alginate, potassium alginate, natural gums, synthetic gums, gum arabic, gellan gum, welan gum, gum tragacanth, xanthan gum, guar gum, locust bean gum, celluloses, carboxymethyl cellulose, hydroxypropyl cellulose, hydroxymethyl cellulose, hydroxybutyl carboxymethyl cellulose, hydroxypropylethyl cellulose and methylethyl cellulose, pullulan, carrageenans, and combinations thereof.

4. A microcapsule according to Claim 1, **characterized in that** said mineral salt is a divalent mineral salt.

5. A microcapsule according to Claim 4, **characterized in that** said divalent mineral salt is selected from the group comprising tricalcium phosphate, calcium hydrogen phosphate, calcium pyrophosphate, calcium carbonate, coral calcium, calcium hydroxyapatite, calcium sulfate, calcium citrate, calcium chloride, calcium malate, calcium lactate, calcium ascorbate, calcium acetate, calcium succinate, calcium piruvate, calcium citrate-malate, calcium gluconate, calcium lactate-gluconate, calcium glubionate, calcium glycerylphosphate, calcium glucoheptonate, calcium pangamate, magnesium sulfate, magnesium phosphate, magnesium pyrophosphate, magnesium carbonate, magnesium chloride, magnesium citrate, magnesium tricitrate, magnesium gluconate, magnesium lactate, magnesium aspartate, magnesium levulinate, magnesium pidolate, magnesium ororate, and combinations thereof.

6. A microcapsule according to Claim 1, **characterized in that** the amount of said mineral salt ranges from 0.01 to 60% of the total weight of the microcapsule.

7. A microcapsule according to Claim 1, **characterized in that** said edible oil of the emulsion is selected from the group comprising vegetable oils, soybean oil, palm kernel oil, palm oil, rapeseed oil, safflower oil, flaxseed oil, evening-primrose oil, sesame oil, sunflower seed oil, canola oil, cottonseed oil, babassu oil, olive oil, algae oils, animal-derived fats, lard, tallow, marine oils, fish oils, sardine oil, cod liver oil, menhaden oil, salmon oil, herring oil, mackerel oil , anchovies oil, edible reduced-fats, low-calorie fats, non-digestible fats, fat-substitutes, synthetic fats, sucrose polyesters, naturally occurring triglycerides derived from vegetable sources, synthetic triglycerides of fatty acids, natural triglycerides of fatty acids, commercial mixtures of alkyl esters or triglycerides of polyunsaturated fatty acids (PUFA), medium chain triglycerides oil (MCT oil), caprylic/capric triglyceride, glyceryl tricaprylate/caprate, corn oil, peanut oil, glycerol monooleate, their partially hydrogenated derivatives and mixtures thereof.

8. A microcapsule according to Claim 1, **characterized in that** said microcapsule comprises at least one plastizicer.

9. A microcapsule according to Claim 1, **characterized in that** said microcapsule comprises at least one wax in the matrix and/or as an outer coating.

10. A coacervation process for forming stable microcapsules according to Claim 1, comprising the formation and the recovery of said microcapsules in an oil bath.

11. A fortified food product containing the microcapsules of Claim 1.

12. A fortified food product according to Claim 11, which comprises from 0.5% to 50% by weight of said microcapsules.

13. A fortified food product according to Claim 11, **characterized in that** said fortified food product is selected from the group comprising jelly-type confections, fruit and vegetable preserves, dairy products, milks, infant formulas, yogurts, cheeses, butters, margarines, milk shakes and ice creams, soy products, soy milks, soy yogurts, soy cheeses, vegetable milks, almond milk, rice milk, beverages, syrups, desserts, puddings, custards, pasty foods, creamy foods and confectionary formulations.

14. A fortified food product according to Claim 11, **characterized in that** said fortified food product comprises 100% Recommended Daily Allowance of mineral requirements for an adult, per unit portion of said fortified food product.

15. Use of the stable microcapsules according to Claim 1 in the preparation of a fortified food product for the treatment and/or prevention of bone demineralization, osteoporosis or extra calcium requirements.

## Amended claims

### Amended claims in accordance with Rule 137(2) EPC.

**1.** A stable microcapsule comprising a protein-polysaccharide shell matrix and a core, said core comprising an emulsion of an aqueous dispersion or solution of at least one mineral salt in an edible oil, said mineral salt being entrapped in the core of the microcapsule set apart from the protein-polysaccharide matrix.

**2.** A microcapsule according to Claim 1, **characterized in that** the protein of said shell matrix is selected from the group comprising gelatin, caseins and caseinates, whey proteins, proteins from legumes and tubers, soy proteins, pea proteins, wheat proteins, corn proteins, barley proteins, egg proteins, muscle proteins, glycoproteins, chondroitins, glucosaminoglycans, lectins, and combinations thereof.

**3.** A microcapsule according to Claim 1, **characterized in that** the polysaccharide of said shell matrix is selected from the group comprising agar, fructo-oligosaccharides, inulin, modified starches, natural starches, starch fractions, amylase, amylopectin, pectins, low methoxy pectins, high methoxy pectins, alginates, sodium alginate, potassium alginate, natural gums, synthetic gums, gum arabic, gellan gum, welan gum, gum tragacanth, xanthan gum, guar gum, locust bean gum, celluloses, carboxymethyl cellulose, hydroxypropyl cellulose, hydroxymethyl cellulose, hydroxybutyl carboxymethyl cellulose, hydroxypropylethyl cellulose and methylethyl cellulose, pullulan, carrageenans, and combinations thereof.

**4.** A microcapsule according to Claim 1, **characterized in that** said mineral salt is a divalent mineral salt.

**5.** A microcapsule according to Claim 4, **characterized in that** said divalent mineral salt is selected from the group comprising tricalcium phosphate, calcium hydrogen phosphate, calcium pyrophosphate, calcium carbonate, coral calcium, calcium hydroxyapatite, calcium sulfate, calcium citrate, calcium chloride, calcium malate, calcium lactate, calcium ascorbate, calcium acetate, calcium succinate, calcium piruvate, calcium citrate-malate, calcium gluconate, calcium lactate-gluconate, calcium glubionate, calcium glycerylphosphate, calcium glucoheptonate, calcium pangamate, magnesium sulfate, magnesium phosphate, magnesium pyrophosphate, magnesium carbonate, magnesium
